# EUROPÄISCHE PATENTSCHRIFT

(11) **EP 2 522 385 B1**
(45) Veröffentlichungstag und Bekanntmachung des Hinweises auf die Patenterteilung: **02.04.2014**
(21) Anmeldenummer: 12164377.9
(22) Anmeldetag: 17.04.2012
(51) Int. Cl.: A61M 25/00, A61F 2/95

(54) **Elastische Kappe für den Schutz des distalen Endes eines Katheters mit Innen- und Außenschlauch**
Elastic cap for the protection of the distal end of a catheter having an inner and an outer hose
Clapet élastique pour la protection de l'extrémité distale d'un cathéter dotée d'un tuyau flexible intérieur et extérieur

(30) Priorität: 10.05.2011 US 201161484247 P
(43) Veröffentlichungstag der Anmeldung: 14.11.2012
(73) Patentinhaber: Biotronik AG, 8180 Bülach (CH)
(72) Erfinder: Fargahi, Amir, 8180 Bülach (CH)
(74) Vertreter: Lindner-Vogt, Karin L.

(56) Entgegenhaltungen:
- FR-A1- 2 707 505
- US-A1- 2006 173 422
- US-A1- 2006 259 120
- US-A1- 2010 069 852
- US-A1- 2010 160 863

## Beschreibung

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen. Stents herkömmlicher Bauart weisen dazu einen rohrförmigen Grundkörper mit einer filigranen Tragstruktur aus metallischen Streben auf, der zur Einbringung in den Körper zunächst in einer komprimierten Form vorliegt und am Ort der Applikation aufgeweitet wird. Zur Platzierung von Stents werden in der Regel Katheter mit einem Innen- und einem Außenschlauch als Applikationsvorrichtung verwendet. Dabei können unterschiedliche Katheter mit Innen- und Außenschlauch genutzt werden.

Zur Applikation nicht selbstexpandierbarer Stents werden meist Ballonkatheter verwendet. Dabei wird der Stent auf eine Oberfläche des auf dem Innenschlauch des Katheters befindlichen nicht-dilatierten Ballons, meist durch sogenanntes "crimpen", fixiert und durch Überlagerung mit einem Außenschlauch vor Beschädigungen während der Positionierung des Katheters geschützt. Nachdem der mit dem Stent beladene Ballonkatheter in das Gefäß eingeführt wurde und der Stent an der Stelle angelangt ist, an der er appliziert werden soll, wird der Außenschlauch zurückgezogen und der Ballon dilatiert. Dadurch wird der Stent geweitet und mit der Gefäßwand in Kontakt gebracht. Nachdem der Stent ausreichend geweitet und das Gefäß ausreichend aufgedehnt wurde, wird der Druck im Ballon verringert, der Ballon kehrt in einen nicht-dilatierten Zustand zurück und der Katheter kann wieder entfernt werden, während der applizierte Stent im Gefäß verbleibt und dieses offen hält. Geeignete Ballonkatheter zur Stentapplikation sind bekannt und beispielhaft in DE 102 15 462 beschrieben.

Alternativ kann ein selbstexpandierendes Stentdesign verwendet werden. Applikationsvorrichtungen zur Applikation selbstexpandierender Stents weisen in der Regel keinen dilatierbaren Ballon zur Stentapplikation auf. Hier werden meist Kathetervorrichtungen verwendet, die einen Innenschlauch und einen Außenschlauch aufweisen, wobei der zu applizierende Stent zunächst auf einer Oberfläche des Innenschlauchs fixiert oder gecrimpt vorliegt und von einem darüber geschobenen Außenschlauch bedeckt ist. Selbstexpandierende Stents weisen in gecrimpter Form eine radiale Kraft auf, die die Stentaußenfläche an die den Stent abdeckende Innenfläche des Außenschlauchs drückt. Der Katheter wird in das Gefäß eingeführt, bis der Stent an der Stelle zu liegen kommt, an der er appliziert werden soll. Zur Applikation wird nun der Außenschlauch zurückgezogen, bis der gesamte Stent freigelegt ist. Durch die radiale Kraft expandiert der Stent eigenständig, löst sich vom Innenschlauch und tritt mit der Gefäßwand in Kontakt. Nachdem sich der Stent ausreichend geweitet hat, kann der Katheter wieder entfernt werden, während der applizierte Stent im Gefäß verbleibt und dieses offen hält. Geeignete Katheter zur Applikation von selbstexpandierbaren Stents sind bekannt und beispielhaft in US 5,824,041 beschrieben.

US 2006/0173422 beschreibt eine Einführvorrichtung mit einem Mechanismus zur Fixierung einer Hülse. US 2010/160863 beschreibt ein Einführsystem für einen Katheter mit einer Katheterhülle mit einem Ventil, welches eine Reibungsverbindung mit der Umgebung herstellt. FR 2 707 505 beschreibt einen Katheter mit einem Katheterschlauch und mindestens zwei 2-Wege-Ventilen. US 2010/0069852 beschreibt einen Katheter mit einer Hülle, die an ihren gegenüberliegenden Enden mit einem konzentrischen Rohr verbunden ist. US 2006/0259120 beschreibt ein Einführsystem für ein medizinisches Implantat, welches die Orientierung zwischen dem Führungsdrahtlumen eines inneren Mittels des Systems und einem Rapid-Exchange Port eines äußeren Mittels erhält.

Ein Problem dieser Katheter mit einem Innen- und einem Außenschlauch ist, dass, während der Positionierung des Katheters im Gefäßsystem des Patienten, unkontrollierte und zum Teil hohe Belastungen auf das distale Ende des Innen- und Außenschlauchs einwirken. Dies kann insbesondere dazu führen, dass die Schlauchwand am distalen Ende des Außenschlauchs irreversibel gedehnt wird und ein sog. "fish mouth" ausgebildet wird. Diese "fish mouth"-Bildung kann sowohl während der Applikation des Katheters als auch beim Entfernen des Katheters zu Verletzungen der Gefäßwand beitragen sowie zu Fehlfunktionen während der Katheterpositionierung führen.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung Mittel bereitzustellen, die einen verbesserten Schutz des distalen Endes eines Katheters mit einem Innen- und einem Außenschlauch erlauben.

Zur Lösung dieser Aufgabe ist ein Katheter entsprechend dem Anspruch 1 ausgebildet.

Die vorliegende Erfindung löst diese Aufgabe durch Bereitstellung einer elastischen Kappe für die Abdeckung des distalen Endes eines Katheters mit Innen- und Außenschlauch. Die elastische Kappe zeichnet sich dadurch aus, dass die Kappe aus einem elastischen Material besteht; eine Kavität aufweist, in die das distale Ende des Innen- und des Außenschlauchs eines Katheters aufnehmbar ist; und die Kappe eine erste Öffnung aufweist, über die die Kavität von außen zugänglich ist, wobei die erste Öffnung vor der Aufnahme des distalen Endes des Innenschlauchs einen Durchmesser aufweist, der nicht größer ist, bevorzugt kleiner ist, als der Außendurchmesser des Innenschlauchs des Katheters und wobei die erste Öffnung elastisch dehnbar ist auf einen Durchmesser, der dem Außendurchmesser des Außenschlauchs des Katheters entspricht.

Wird die elastische Kappe auf das distale Ende eines Katheters appliziert, so wird sowohl der Innenschlauch als auch der Außenschlauch im Bereich des distalen Endes von der Kappe bedeckt. Das distale Ende des Innen und des Außenschlauchs des Katheters werden durch die erste Öffnung hindurch in die Kavität der elastischen Kappe eingeführt. Die erste Öffnung ist dabei an dem Ende der erfindungsgemäßen Kappe angeordnet, welches entgegen der während der Katherapplikation verwendeten Schubrichtung gelegen ist. Beim Anbringen der erfindungsgemäßen Kappe wird die erste Öffnung mindestens soweit elastisch aufgeweitet, dass die Innenoberfläche der Kavität in einem den Rand der ersten Öffnung umfassenden Bereich mit der Außenoberfläche des Außenschlauchs in Kontakt kommt und die Kappe somit mit dem Außenschlauch überlappt. Der Katheter kann nun gefahrlos appliziert werden.

Hat das distale Ende des Katheters den Bestimmungsort im Patienten erreicht, wird der Außenschlauch zurückgezogen und das distale Ende des Außenschlauchs verlässt die Kavität der Kappe. Sobald das distale Ende des Außenschlauchs die erste Öffnung passiert hat und damit aus der erfindungsgemäßen Kappe entfernt wurde, verengt sich der Durchmesser der ersten Öffnung aufgrund der elastischen Eigenschaften der erfindungsgemäßen Kappe und die Kappe legt sich in einem Bereich umfassend den Rand der ersten Öffnung an die Außenoberfläche des Innenschlauchs an. Im Bereich der ersten Öffnung entsteht somit ein stufenloser Übergang vom Innenschlauch zur elastischen Kappe. Der Katheter kann nun, nachdem er seinen Zweck erfüllt hat, wieder entfernt werden, ohne dass dabei das umliegende, gesunde Gewebe über Gebühr belastet wird.

Die Verwendung der erfindungsgemäßen elastischen Kappe bei der Applikation eines Katheters mit einem Innen- und einem Außenschlauch stellt sicher, dass während der Applikation des Katheters die Flächen am distalen Ende des Innen- und Außenschlauchs des Katheters vor direktem Kontakt mit der Umgebung geschützt sind. Das distale Ende des Außenschlauchs wird nicht deformiert und es bildet sich kein sog. "fish mouth" aus. Somit wird die Belastung des Patienten während der Applikation des Katheters vermindert, die Gefahr von ungewollten Beeinträchtigungen von gesundem Gewebe wird minimiert. Damit nimmt auch insgesamt die Belastung ab, die während der Applikation auf das distale Ende des Katheters einwirkt. Als Folge nehmen auch die Probleme während der Katheterapplikation ab, wie beispielsweise ein mögliches Verschieben des Außenschlauchs über das distale Ende des Innenschlauchs hinweg oder Beschädigungen oder Lageveränderungen eines Stents, der mit dem Katheter platziert werden soll. Die Verwendung der erfindungsgemäßen Kappe sorgt auch dafür, dass der Katheter nach bestimmungsgemäßem Gebrauch gefahrlos wieder entfernt werden kann, da sich die Kappe aufgrund ihrer elastischen Eigenschaften des Kappenmaterials nach der Entfernung des Außenschlauchs unmittelbar an den Innenschlauch anlegt und somit die Ausbildung von ggf. störenden Hindernissen wie z.B. Stufen oder Graten verhindert, welche beim herausziehen des Katheters zu Verletzungen führen könnten.

Die erfindungsgemäße Kappe weist eine äußerliche Grundform auf, die, nach dem Anbringen der Kappe auf dem distalen Ende eines Katheters, die Einführung und Bewegung des Katheters im Gefäßbett eines Patienten erlaubt ohne das umliegende Gewebe über Gebühr zu strapazieren. Dazu kann die Kappe eine Grundform aufweisen, die entlang der Längsachse der Kappe gestreckt ist, also eine längliche Grundform. Die Grundform kann radialsymmetrisch sein. Die erfindungsgemäße elastische Kappe kann sich dadurch auszeichnen, dass das der ersten Öffnung gegenüberliegende Ende der Kappe als stumpfe Spitze ausgebildet ist. Am der ersten Öffnung gegenüberliegenden Ende der erfindungsgemäßen Kappe kann eine zweite Öffnung vorgesehen sein, die derart ausgeführt sein kann, dass dadurch ein Führungsdraht für einen Katheter führbar ist. Die Außenoberfläche der Kappe kann glatt ausgebildet sein oder strukturiert. Die Außenoberfläche der Kappe kann beschichtet sein mit einer oder mehrerer Schichten eines oder mehrerer Materialien. Die Beschichtung kann beispielsweise dem Schutz der Kappe dienen, die Beschichtung kann auch röntgensichtbare Materialien aufweisen z.B. zur Darstellung der Kappe im Körper eines Patienten oder die Beschichtung kann zur Vermeidung von Wechselwirkungen der Kappenoberfläche mit der Umgebung vor oder während der Applikation eines mit der Kappe ausgestatteten Katheters ausgebildet sein.

Die erfindungsgemäße Kappe ist aus einem elastischen Material gefertigt. Bevorzugt besteht die Kappe aus einem im Wesentlichen biokompatiblen und/oder bioinerten Material. Insbesondere kann die erfindungsgemäße Kappe aus einem elastischen Material bestehen, welches Silikone, Polyamide (PA), Copolyamide, Polyether-Blockamide (z. B. solche mit dem Markennamen PEBAX), Polyurethane (PUR) und/oder eine Mischung davon umfasst oder daraus besteht. Um die Sichtbarkeit der Kappe während der Applikation eines damit versehenen Katheters zu ermöglichen, kann dem elastischen Material der erfindungsgemäßen Kappe beispielsweise röntgensichtbares Material beigemischt sein.

Die erfindungsgemäße Kappe kann durch herkömmliche Verfahren hergestellt sein z.B. durch Extrusions-, Spritz-, Guss- und/oder Spritzgussverfahren. Geeignete Verfahren sind dem Fachmann bekannt.

Dem Fachmann ist bewusst, dass die erfindungsgemäße Kappe in ihren Dimensionen auf einen damit zu verwendenden Kathetertyp abgestimmt sein muss. Die Angabe von absoluten Dimensionen ist bei der Definition der Kappe wenig hilfreich. Der Fachmann ist sich darüber klar, dass die Dimensionen der Kappe nur in Bezug auf einen ggf. damit zu verwendenden Katheter sinnvoll definiert werden können, ohne dass der entsprechende Katheter Bestandteil der Ansprüche sein muss. Ausgehend von einem bestimmten Katheter und den in dieser Beschreibung bereitgestellten Angaben ist der Fachmann ohne unzumutbaren Aufwand in der Lage eine erfindungsgemäße Kappe herzustellen, die die genannten Eigenschaften aufweist.

Die erfindungsgemäße Kappe weist eine Kavität auf, die derart ausgebildet ist, dass darin sowohl das distale Ende des Innenschlauchs eines Katheters als auch das distale Ende des Außenschlauchs des Katheters aufgenommen werden können. Dabei wird unter dem distalen Ende des Innen- und Außenschlauchs jeweils das freie Ende des Schlauchteils verstanden, der während der Applikation des Katheters in den Körper des Patienten eingeführt wird. Das distale Ende umfasst dabei mindestens den unmittelbaren Randbereich um das freie Schlauchende einschließlich der frontalen Außenoberfläche und mindestens einem Teil der angrenzenden seitlichen Außenoberfläche des betreffenden Schlauchteils.

Die Kavität der erfindungsgemäßen Kappe kann mehrere unterschiedliche Bereiche aufweisen. Beispielsweise kann die Kavität einen ersten Bereich umfassen, der der Aufnahme des distalen Endes des Innenschlauchs dient und dazu derart ausgestaltet ist, dass in diesem Bereich die Innenoberfläche der Kavität mit der Außenoberfläche am distalen Ende des Innenschlauchs kontaktierbar ist. Dieser erste Bereich kann dabei so ausgebildet sein, dass dieser Bereich geeignet ist das distale Ende des Innenschlauchs aufzunehmen, während das distale Ende des Außenschlauchs in diesen ersten Bereich nicht aufgenommen werden kann. Dies kann beispielsweise dadurch erreicht werden, dass der Durchmesser der Kavität im ersten Bereich derart gewählt ist, dass dieser kleiner ist als der Außendurchmesser des Außenschlauchs und gleichzeitig größer oder gleich dem Außendurchmesser des Innenschlauchs. In einer besonderen Ausführungsform entspricht der Durchmesser der Kavität im ersten Bereich im Wesentlichen dem Außendurchmesser des Innenschlauchs.

Die Kavität der erfindungsgemäßen Kappe kann einen zweiten Bereich aufweisen, welcher zwischen der ersten Öffnung der Kappe und dem ersten Bereich der Kavität angeordnet ist. Dieser zweite Bereich dient der Aufnahme des distalen Endes des Außenschlauchs und ist dazu derart ausgestaltet, dass in diesem Bereich die Innenoberfläche der Kavität mit der Außenoberfläche des distalen Endes des Außenschlauchs kontaktiert werden kann. Dieser zweite Bereich kann dabei so ausgebildet sein, dass dieser Bereich geeignet ist, bevorzugt das distale Ende des Außenschlauchs aufzunehmen. Dies kann beispielsweise dadurch erreicht werden, dass der Durchmesser der Kavität im zweiten Bereich derart gewählt ist, dass dieser größer ist als der Außendurchmesser des Innenschlauchs und gleichzeitig größer oder gleich dem Außendurchmesser des Außenschlauchs. In einer besonderen Ausführungsform entspricht der Durchmesser der Kavität im zweiten Bereich im Wesentlichen dem Außendurchmesser des Außenschlauchs.

In einer bevorzugten Ausführungsform der erfindungsgemäßen Kappe ist der Durchmesser der Kavität im ersten Bereich geringer als der Außendurchmesser des Außenschlauchs und der Durchmesser der Kavität im zweiten Bereich ist größer als im ersten Bereich, besonders bevorzugt entspricht der Durchmesser der Kavität im ersten Bereich im Wesentlichen dem Außendurchmesser des Innenschlauchs und im zweiten Bereich im Wesentlichen dem Außendurchmesser des Außenschlauchs.

In der erfindungsgemäßen Kappe ist die Kavität von außen über eine erste Öffnung zugänglich und das distale Ende eines Katheters mit Innen- und Außenschlauch kann über diese Öffnung in die Kavität der Kappe eingeführt werden. Die erste Öffnung ist an dem Ende der Kappe vorgesehen, welches der gewünschten Schubrichtung während der Applikation des Katheters gegenüber liegt. In einer bevorzugten Ausführungsform weist die erfindungsgemäße Kappe eine stumpfe Spitze auf und die erste Öffnung ist an dem Ende der Kappe vorgesehen, welches der stumpfen Spitze gegenüber liegt.

Die erste Öffnung dient der Aufnahme der distalen Enden des Innen- und Außenschlauchs eines Katheters in die Kavität der Kappe. Dazu ist die erste Öffnung derart ausgeführt, dass sie vor der Aufnahme des distalen Endes des Katheters mit einem Innen- und einem Außenschlauch einen Durchmesser aufweist, der nicht größer ist als der Außendurchmesser des Innenschlauchs des Katheters. Bevorzugt ist der Durchmesser der ersten Öffnung vor Aufnahme des Innenschlauchs kleiner als der Außendurchmesser des Innenschlauchs. Dadurch ist sichergestellt, dass der Randbereich der Kappe im Bereich der ersten Öffnung rundum schlüssig an der Außenoberfläche des Innenschlauchs anliegen kann. Aufgrund der Eigenschaften des elastischen Materials der erfindungsgemäßen Kappe ist die erste Öffnung elastisch dehnbar auf einen Durchmesser, der im Wesentlichen dem Außendurchmesser des Außenschlauchs entspricht. Dadurch ist sichergestellt, dass der Randbereich der Kappe im Bereich der ersten Öffnung rundum schlüssig an der Außenoberfläche des Außenschlauchs anliegen kann. Die erste Öffnung der erfindungsgemäßen Kappe ist also derart elastisch ausgebildet, dass der Randbereich der ersten Öffnung sowohl schlüssig an der Außenoberfläche des Außenschlauchs als auch an der Außenoberfläche des Innenschlauchs anliegen kann. Dies führt dazu, dass zunächst beim Anbringen der Kappe auf dem distalen Ende eines Katheters sowohl der Innen- als auch der diesen umgebenden Au-ßenschlauch in die Kavität der Kappe eingeführt werden können. Nun liegt der Randereich der ersten Öffnung schlüssig an der Außenoberfläche des Außenschlauchs an und der Katheter kann bestimmungsgemäß appliziert werden, wobei die erfindungsgemäße Kappe das distale Ende des Katheters schützt. Sobald der Katheter im Patienten die gewünschte Position erreicht hat, kann der Außenschlauch zurückgezogen werden, der Außenschlauch tritt aus der Kavität aus und verlässt die Kappe, während der Innenschlauch durch die Kappe geschützt bleibt. Der Randbereich der ersten Öffnung schließt sich nach dem Entfernen des Außenschlauchs aufgrund der elastischen Eigenschaften des Kappenmaterials schlüssig um die Außenoberfläche des Innenschlauchs. Dadurch wird sichergestellt, dass sich nach dem Abziehen des Außenschlauchs zwischen der Kappe und dem Innenschlauch keine Stufen oder Grate bilden und der Katheter so problemlos aus dem Patienten entfernt werden kann.

Die Kappe kann insbesondere derart ausgestaltet sein, dass sich das elastische Material noch im linear elastischen Bereich befindet, wenn die erste Öffnung auf einen Durchmesser ausgedehnt ist, der dem Außendurchmesser des Außenschlauchs des Katheters entspricht.

Um sicherzustellen, dass der Randbereich der ersten Öffnung der erfindungsgemäßen Kappe den Außenschlauch nicht beschädigt und ein Herausziehen des Außenschlauchs aus der Kappe ohne zu großen Kraftaufwand gewährleistet, ist es vorteilhaft, wenn der Randbereich eine Kraft auf die Außenoberfläche des Außenschlauchs ausübt, die weder zu groß noch zu klein ist. Insbesondere kann die elastische Kappe derart ausgebildet sein, dass die Öffnung mit einer Kraft von ≤ 2,5 MPa, bevorzugt von 0,5 MPa bis 2,0 MPa, auf einen Durchmesser dehnbar ist, der dem Außendurchmesser des Außenschlauchs eines mit der Kappe zu verwendenden Katheters entspricht. Dabei wird davon ausgegangen, dass sich das elastische Material im elastischen Bereich befindet und die Kraft, die für die Dehnung aufgewendet werden muss im Wesentlichen der Kraft entspricht, mit der der gedehnte Bereich wieder in seinen Ausgangszustand zurückzukehren versucht.

Um sicherzustellen, dass der Randbereich der ersten Öffnung der erfindungsgemäßen Kappe den Innenschlauch nicht beschädigt und dass nach dem Entfernen des Außenschlauchs ein schlüssigen Anliegen des Randbereichs der ersten Öffnung an die Außenoberfläche des Innenschlauchs gewährleistet ist, ist es vorteilhaft, wenn der Randbereich eine Kraft auf die Außenoberfläche des Innenschlauchs ausübt, die weder zu groß noch zu klein ist. Insbesondere kann die elastische Kappe derart ausgebildet sein, dass die Öffnung mit einer Kraft von ≤ 1,5 MPa, bevorzugt von 0,2 MPa bis 0,5 MPa, auf einen Durchmesser dehnbar ist, der dem Außendurchmesser des Innenschlauchs eines mit der Kappe zu verwendenden Katheters entspricht. Dabei wird davon ausgegangen, dass sich das elastische Material im elastischen Bereich befindet und die Kraft, die für die Dehnung aufgewendet werden muss im Wesentlichen der Kraft entspricht, mit der der gedehnte Bereich wieder in seinen Ausgangszustand zurückzukehren versucht.

Die vorliegende Erfindung bezieht sich auch auf einen Katheter mit einem Innen- und einem Außenschlauch, dadurch gekennzeichnet, dass der Katheter an seinem distalen Ende eine erfindungsgemäße elastische Kappe aufweist. Insbesondere kann die erfindungsgemä-ße Kappe derart auf dem Katheter angebracht sein, dass der zweite Bereich der Kavität der elastischen Kappe die Außenoberfläche der Seitenwand des distalen Endes des Außenschlauchs kontaktiert. Damit ist sichergestellt, dass die Kappe das distale Ende des Katheters umschließt und mit dem Außenschlauch überlappt.

Insbesondere kann es sich bei dem Katheter um einen Katheter handeln, der als Applikationsvorrichtung für einen Stent ausgebildet ist. Der erfindungsgemäße Katheter kann zusätzlich zur erfindungsgemäßen elastischen Kappe einen Stent umfassen. Der erfindungsgemäße Katheter kann beispielsweise als Vorrichtung zur Applikation von selbstexpandierbaren Stents ausgestaltet sein. Vorrichtungen zur Applikation selbstexpandierbarer Stents sind dadurch gekennzeichnet, dass keine Mittel zur aktiven plastischen Verformung eines aufgecrimpten Stents notwendig sind, wie z.B. expandierbare Ballons, die üblicherweise auf Ballonkathetern vorgesehen sind. Bevorzugt werden Katheter verwendet, die anstatt eines solchen Ballons einen Bereich eines Innenschlauchs aufweisen, auf den der selbstexpandierbare Stent zur Applikation fixiert werden kann. Zusätzlich weist ein solcher Katheter Mittel auf, die es erlauben den fixierten oder gecrimpten selbstexpandierbaren Stent solange an einer Selbstexpansion zu hindern, bis der Stent an die vorherbestimmte Position gebracht worden ist und dann ein Auslösen der Selbstexpansion des Stents gestatten. Dies kann beispielsweise dadurch erreicht werden, dass der auf den Innenschlauch gecrimpte selbstexpandierbare Stent von einem Außenschlauch derart umgeben ist, dass der Stent an einer Selbstexpansion gehindert ist. Dieser Außenschlauch kann dabei derart ausgestaltet sein, dass ein Zurückziehen des Außenschlauchs im Verhältnis zum Innenschlauch möglich ist, so dass ein Bereich des Innenschlauchs freigesetzt werden kann, auf den der selbstexpandierbare Stent gecrimpt vorliegt. Liegt der Außenschlauch weit genug zurückgezogen vor, so kann der Stent sich aufgrund seiner radialen Kraft vom Innenschlauch lösen, eigenständig expandieren und mit der Gefäßwand in Kontakt treten.

### Figuren

- Fig. 1: zeigt eine erste Ausführungsform der erfindungsgemäßen Kappe.
- Fig. 2: zeigt einen Katheter mit einer erfindungsgemäßen Kappe, wobei die Kappe mit dem Außenschlauch überlappt.
- Fig. 3: zeigt einen Katheter mit einer erfindungsgemäßen Kappe ohne Außenschlauch.

Nachfolgend wird die Erfindung anhand von Ausführungsbeispielen näher erläutert.

### Ausführungsbeispiel 1

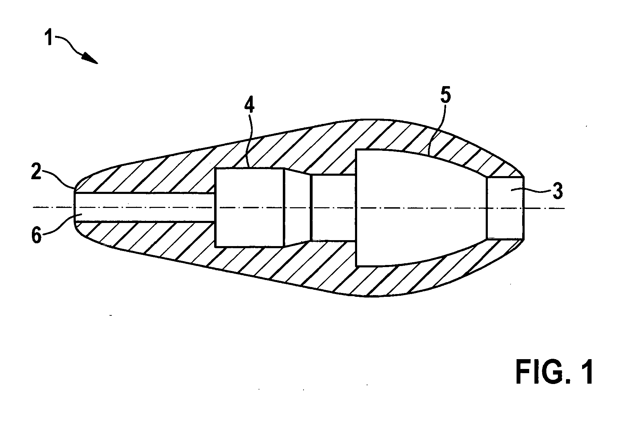

In Figur 1 ist eine Ausführungsform der erfindungsgemäßen elastischen Kappe 1 dargestellt.

Die Kappe 1 ist in einem Spritzverfahren einteilig aus einem elastischen Material gefertigt. Dabei weist die Kappe 1 eine radialsymmetrische, längliche Grundform auf, mit einer stumpfen Spitze 2 an einem ersten Ende der Kappe 1 und einer ersten Öffnung 3 an einem dem ersten Ende gegenüberliegenden, zweiten Ende der Kappe 1. Im Inneren der Kappe liegt eine Kavität, die einen ersten Bereich 4 und einen zweiten Bereich 5 aufweist. Der erste Bereich 4 der Kavität ist derart ausgeführt und dimensioniert, dass darin das distale Ende des Innenschlauchs eines Katheters aufgenommen werden kann. Ist die Kappe auf dem distalen Ende eines Katheters mit Innen- und Außenschlauch positioniert, so kontaktiert die Innenfläche der Kavität im ersten Bereich 4 die Außenoberfläche des Innenschlauchs. Der zweite Bereich 5 der Kavität ist derart ausgestaltet und dimensioniert, dass darin das distale Ende des Außenschlauchs eines Katheters aufgenommen werden kann. Ist die Kappe 1 auf dem distalen Ende des Katheters positioniert, so kontaktiert die Innenfläche der Kavität im zweiten Bereich 5 die Außenoberfläche des Außenschlauchs.

Die erste Öffnung 3 ist derart ausgeführt, dass darüber die Kavität von außen zugänglich ist. Die Öffnung 3 weist vor Aufnahme des distalen Endes eines Katheters einen Durchmesser auf, der nicht größer ist als der Außendurchmesser des Innenschlauchs des Katheters. Die erste Öffnung 3 ist derart elastisch dehnbar ausgestaltet, dass die Öffnung 3 auf einen Durchmesser dehnbar ist, der dem Außendurchmesser des Außenschlauchs des Katheters entspricht.

Gegenüber der ersten Öffnung 3 weist die Kappe 1 eine zweite Öffnung 6 auf, durch die der Führungsdraht eines Katheters geführt werden kann, so dass der Katheter nach Anbringung der Kappe 1 an seinem distalen Ende weiterhin über seinen Führungsdraht steuerbar ist.

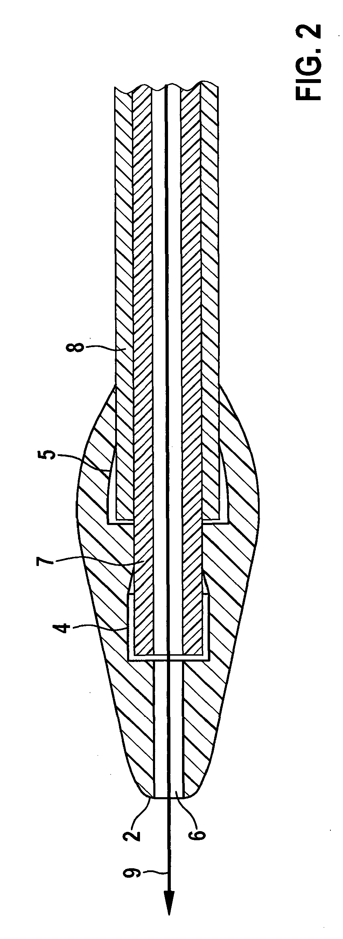

In Figur 2 ist ein Katheter mit einer erfindungsgemäßen Kappe 1 aus Figur 1 gezeigt. Die Kappe 1 bedeckt das distale Ende des Katheters, wobei die Kappe 1 sowohl mit dem Innenschlauch 7 als auch mit dem Außenschlauch 8 des Katheters überlappt. Der Führungsdraht 9 ist durch die zweite Öffnung 6 geführt und kann zur Steuerung des Katheters während der Applikation im Patienten verwendet werden. Das distale Ende des Innenschlauchs 7 kommt im ersten Bereich 4 der Kavität der Kappe 1 zu liegen, während das distale Ende des Außenschlauchs 8 im zweiten Bereich 5 der Kavität der Kappe 1 positioniert ist. Sowohl der Innenschlauch 7 als auch der Außenschlauch 8 ragen durch die erste Öffnung 3 in die Kavität der Kappe 1. Dabei liegt der Randbereich der Kappe 1 im Bereich der ersten Öffnung 3 an der Außenoberfläche des Außenschlauchs rundum schlüssig an. Das distale Ende des Katheters liegt durch die Kappe 1 vollständig geschützt vor und der Katheter kann problemlos appliziert werden.

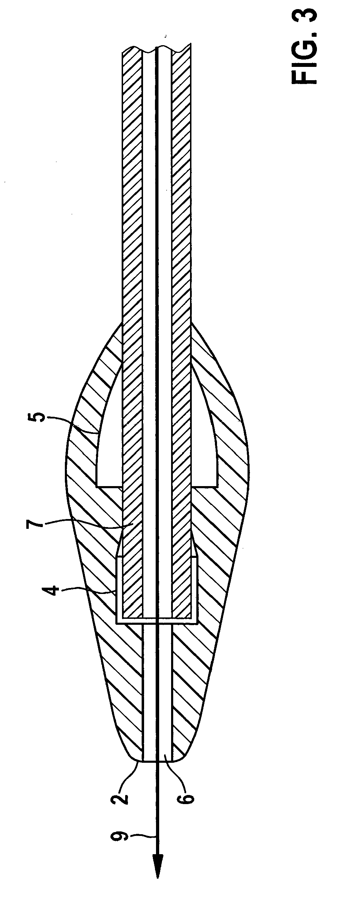

In Figur 3 ist der Katheter aus Figur 2 dargestellt, wobei der Außenschlauch 8 entfernt wurde. Nachdem der Außenschlauch 8 zurückgezogen und aus dem distalen Endbereich des Katheters entfernt wurde, hat das distale Ende des Außenschlauchs die Kappe 1 verlassen und der Randbereich der Kappe 1 im Bereich der ersten Öffnung 3 hat sich, aufgrund der elastischen Eigenschaften des Kappenmaterials, an die Außenoberfläche des Innenschlauchs 7 rundum schlüssig angelegt. Das distale Ende des Katheters liegt weiterhin durch die Kappe 1 vollständig geschützt vor und der Katheter kann problemlos entfernt werden.

## Patentansprüche

1. Katheter mit einem Innen- und einem Außenschlauch und einer elastische Kappe (1) an seinem distalen Ende für die Abdeckung des distalen Endes des Katheters mit Innen- (7) und Außenschlauch (8), wobei die Kappe aus einem elastischen Material besteht; eine Kavität aufweist, in die das distale Ende des Innen- (7) und des Außenschlauchs (8) eines Katheters aufnehmbar ist; und die Kappe (1) eine erste Öffnung (3) aufweist, über die die Kavität von außen zugänglich ist, wobei die erste Öffnung (3) vor der Aufnahme des distalen Endes des Katheters einen Durchmesser aufweist, der nicht größer ist als der Außendurchmesser des Innenschlauchs (7) und wobei die erste Öffnung (3) elastisch dehnbar ist auf einen Durchmesser, der dem Außendurchmesser des Außenschlauchs (8) entspricht, **dadurch gekennzeichnet, dass** die Kavität der Kappe (1) einen ersten Bereich (4) aufweist, in dem die Innenoberfläche der Kavität mit dem distalen Ende des Innenschlauchs (7) kontaktierbar ist, und einen zweiten Bereich (5), in dem die Innenoberfläche der Kavität mit dem distalen Ende des Außenschlauchs (8) kontaktierbar ist, wobei der zweite Bereich (5) zwischen dem ersten Bereich (4) und der ersten Öffnung (3) angeordnet ist, und
der Durchmesser der Kavität im ersten Bereich (4) geringer ist als der Außendurchmesser des Außenschlauchs (8), und der Durchmesser im zweiten Bereich (5) größer ist als im ersten Bereich (4).

2. Katheter nach Anspruch 1, **dadurch gekennzeichnet, dass** die Kappe (1) entlang ihrer Längsachse eine längliche Grundform aufweist.

3. Katheter nach einem der vorhergehenden Ansprüche, **dadurch gekennzeichnet, dass** die Grundform der Kappe (1) entlang der Längsachse radialsymmetrisch ist.

4. Katheter nach einem der vorhergehenden Ansprüche, **dadurch gekennzeichnet, dass** das der ersten Öffnung (3) gegenüberliegende Ende der Kappe (1) als stumpfe Spitze (2) ausgebildet ist.

5. Katheter nach einem der vorhergehenden Ansprüche, **dadurch gekennzeichnet, dass** die Kappe (1) auf der der ersten Öffnung (3) gegenüberliegenden Seite eine zweite Öffnung (6) aufweist, bevorzugt ist die zweite Öffnung (6) derart ausgestaltet, dass durch diese Öffnung ein Führungsdraht (9) eines Katheters führbar ist.

6. Katheter nach einem der vorhergehenden Ansprüche, **dadurch gekennzeichnet, dass** die Kappe (1) aus einem elastischen Material besteht, welches Silikone, Polyamide (PA), Copolyamide, Polyether-Blockamide (PEBAX), Polyurethane (PUR) und/oder eine Mischung davon umfasst oder daraus besteht.

7. Katheter nach einem der vorhergehenden Ansprüche, **dadurch gekennzeichnet, dass** die Kappe (1) derart ausgestaltet ist, dass sich das elastische Material bei einer Dehnung der ersten Öffnung (3) auf einen Durchmesser, der dem Außendurchmesser des Außenschlauchs (8) des Katheters entspricht, im linear elastischen Bereich befindet.

8. Katheter nach einem der vorhergehenden Ansprüche, **dadurch gekennzeichnet, dass** die Kappe (1) derart ausgeführt ist, dass die Öffnung mit einer Kraft von ≤ 2,5 MPa auf einen Durchmesser dehnbar ist, der dem Außendurchmesser des Außenschlauchs (8) eines Katheters entspricht.

9. Katheter nach einem der vorhergehenden Ansprüche, **dadurch gekennzeichnet, dass** die Kappe (1) derart ausgeführt ist, dass die Öffnung mit einer Kraft von ≤1,5 MPa auf einen Durchmesser dehnbar ist, der dem Außendurchmesser des Innenschlauchs (7) eines Katheters entspricht.

10. Katheter nach einem der vorhergehenden Ansprüche, **dadurch gekennzeichnet, dass** der zweite Bereich (5) der Kavität der elastischen Kappe (1) die Außenoberfläche des Außenschlauchs (8) kontaktiert.

11. Katheter nach einem der vorhergehenden Ansprüche, **dadurch gekennzeichnet, dass** der Katheter als Applikationsvorrichtung für einen Stent ausgebildet ist.

12. Katheter nach einem der vorhergehenden Ansprüche, **dadurch gekennzeichnet, dass** der Katheter einen Stent umfasst.

## Claims

1. A catheter comprising an inner hose and an outer hose, and comprising an elastic cap (1) on its distal end for covering the distal end of the catheter having an inner hose (7) and an outer hose (8), wherein said cap consists of an elastic material, has a cavity in which the distal end of the inner (7) and the outer hoses (8) of a catheter can be received, and the cap (1) has a first opening (3) via which the cavity is accessible from the outside, wherein prior to receiving the distal end of the catheter, the first opening (3) has a diameter which is not larger than the outer diameter of the inner hose (7), and wherein the first opening (3) is elastically expandable to a diameter which corresponds to the outer diameter of the outer hose (8), **characterized in that** the cavity of the cap (1) has a first region (4) in which the inner surface of the cavity can be brought in contact with the distal end of the inner hose (7), and a second region (5) in which the inner surface of the cavity can be brought in contact with the distal end of the outer hose (8), wherein the second region (5) is arranged between the first region (4) and the first opening (3), and
the diameter in the first region (4) of the cavity is smaller than the outer diameter of the outer hose (8), and the diameter in the second region(5) is larger than in the first region (4).

2. The catheter according to claim 1, **characterized in that** along its longitudinal axis, the cap (1) has an elongated basic shape.

3. The catheter according to any one of the preceding claims, **characterized in that** along the longitudinal axis, the basic shape of the cap (1) is radially symmetric.

4. The catheter according to any one of the preceding claims, **characterized in that** the cap's (1) end located opposite to the first opening (3) is formed as a blunt tip (2).

5. The catheter according to any one of the preceding claims, **characterized in that** on the side opposite to the first opening (3), the cap (1) has a second opening (6), wherein said second opening (6) is preferably configured in such a manner that a guide wire (9) of a catheter can be guided therethrough.

6. The catheter according to any one of the preceding claims, **characterized in that** the cap (1) consists of an elastic material which comprises or consists of silicones, polyamides (PA), copolyamides, polyether block amides (PEBAX), polyurethanes (PUR) and/or a mixture thereof.

7. The catheter according to any one of the preceding claims, **characterized in that** the cap (1) is configured in such a manner that during an expansion of the first opening (3) to a diameter corresponding to the outer diameter of the outer hose (8) of the catheter, the elastic material is in the linear-elastic range.

8. The catheter according to any one of the preceding claims, **characterized in that** the cap (1) is configured in such a manner that the opening can be expanded with a force of ≤ 2.5 MPa to a diameter which corresponds to the outer diameter of the outer hose (8) of a catheter.

9. The catheter according to any one of the preceding claims, **characterized in that** the cap (1) is configured in such a manner that the opening can be expanded with a force of ≤ 1.5 MPa to a diameter which corresponds to the outer diameter of the inner hose (7) of a catheter.

10. The catheter according to any one of the preceding claims, **characterized in that** the second region (5) of the cavity of the elastic cap (1) contacts the outer surface of the outer hose (8).

11. The catheter according to any one of the preceding claims, **characterized in that** the catheter is configured as an application device for a stent.

12. The catheter according to any one of the preceding claims, **characterized in that** the catheter comprises a stent.

## Revendications

1. Cathéter avec un tube intérieur et un tube extérieur et un capuchon élastique (1) à son extrémité distale, pour recouvrir l'extrémité distale du cathéter avec le tube intérieur (7) et le tube extérieur (8), dans lequel le capuchon est constitué d'un matériau élastique ; comporte une cavité, dans laquelle l'extrémité distale du tube intérieur (7) et du tube extérieur (8) d'un cathéter peut être admise ; et le capuchon (1) comporte une première ouverture (3), par laquelle la cavité est accessible depuis l'extérieur, dans lequel la première ouverture (3) présente un diamètre pas plus grand que le diamètre extérieur du tube intérieur (7) avec l'admission de l'extrémité distale du cathéter, et dans lequel la première ouverture (3) peut être élastiquement élargie jusqu'à un diamètre correspondant au diamètre extérieur du tube extérieur (8), **caractérisé en ce que** la cavité du capuchon (1) comporte une première région (4) dans laquelle la surface intérieure de la cavité peut être mise en contact avec l'extrémité distale du tube intérieur (7), ainsi qu'une deuxième région (5) dans laquelle la surface intérieure de la cavité peut être mise en contact avec l'extrémité distale du tube extérieur (8), dans lequel la deuxième région (5) est agencée entre la première région (4) et la première ouverture (3), et
le diamètre de la cavité dans la première région (4) est inférieur au diamètre extérieur du tube extérieur (8), et le diamètre dans la deuxième région (5) est supérieur à celui de la première région (4).

2. Cathéter selon la revendication 1, **caractérisé en ce que** le long de son axe longitudinal, le capuchon (1) présente une forme de base allongée.

3. Cathéter selon l'une des revendications précédentes, **caractérisé en ce que** la forme de base du capuchon (1) est radialement symétrique le long de l'axe longitudinal.

4. Cathéter selon l'une des revendications précédentes, **caractérisé en ce que** l'extrémité du capuchon (1) opposée à la première ouverture (3) est conçue comme une pointe émoussée (2).

5. Cathéter selon l'une des revendications précédentes, **caractérisé en ce que** le capuchon (1) comporte une deuxième ouverture (6) du côté opposé à la première ouverture (3), la deuxième ouverture (6) étant de préférence conçue de manière à ce qu'un fil de guidage (9) d'un cathéter puisse être inséré à travers cette ouverture.

6. Cathéter selon l'une des revendications précédentes, **caractérisé en ce que** le capuchon (1) est constitué d'un matériau élastique comprenant ou étant constitué de silicones, de polyamides (PA), de copolyamides, d'amides-bloc de polyéther (PEBAX), de polyuréthanes (PUR) et/ou d'un mélange de ceux-ci.

7. Cathéter selon l'une des revendications précédentes, **caractérisé en ce que** le capuchon (1) est conçu de manière à ce que le matériau élastique se trouve dans la région linéairement élastique lors d'un élargissement de la première ouverture (3) jusqu'à un diamètre correspondant au diamètre extérieur du tube extérieur (8) du cathéter.

8. Cathéter selon l'une des revendications précédentes, **caractérisé en ce que** le capuchon (1) est conçu de manière à ce que l'ouverture puisse être élargie avec une force ≤ 2,5 MPa jusqu'à un diamètre correspondant au diamètre extérieur du tube extérieur (8) d'un cathéter.

9. Cathéter selon l'une des revendications précédentes, **caractérisé en ce que** le capuchon (1) est conçu de manière à ce que l'ouverture puisse être élargie avec une force ≤ 1,5 MPa jusqu'à un diamètre correspondant au diamètre extérieur du tube intérieur (7) d'un cathéter.

10. Cathéter selon l'une des revendications précédentes, **caractérisé en ce que** la deuxième région (5) de la cavité du capuchon élastique (1) touche la surface extérieure du tube extérieur (8).

11. Cathéter selon l'une des revendications précédentes, **caractérisé en ce que** le cathéter est conçu comme un dispositif d'application pour un stent.

12. Cathéter selon l'une des revendications précédentes, **caractérisé en ce que** le cathéter comprend un stent.
